# EUROPEAN PATENT APPLICATION

(11) **EP 4 533 953 A1**
(43) Date of publication of application: **09.04.2025**
(21) Application number: 23842234.9
(22) Date of filing: 14.07.2023
(51) Int. Cl.: A23C 9/152, A23C 9/154

(54) **MODIFIED MILK HAVING YEAST EXTRACT CONTAINING GAMMA-AMINOBUTYRIC ACID AND PREPARATION METHOD THEREFOR**

(30) Priority: 20.07.2022 CN 202210861656
(71) Applicant: Angel Yeast Co., Ltd, Yichang, Hubei 443003 (CN)
(72) Inventor: QIN, Xianwu, Hubei 443003 (CN); LI, Zhigang, Hubei 443003 (CN); ZHANG, Yan, Hubei 443003 (CN); LI, Pei, Hubei 443003 (CN); LI, Ku, Hubei 443003 (CN); WU, Yexu, Hubei 443003 (CN); YAN, Dongfang, Hubei 443003 (CN); XU, Huai, Hubei 443003 (CN)
(74) Representative: karo IP
(86) International application number: PCT/CN2023/107574
(87) International publication number: WO 2024/017171

(57) **Abstract**

Provided are a modified milk having a yeast extract containing gamma-aminobutyric acid and a preparation method therefor. The modified milk comprises, in a total of 1,000 parts by weight of ingredients: 800-970 parts of raw milk, 0.1-10 parts of a yeast extract containing gamma-aminobutyric acid, 0-4 parts of a thickening agent, 0-3 parts of an emulsifier, 0-1 parts of an acidity regulator, 0-130 parts of a sweetener, and 0-2 parts of a flavor essence, with the balance being water, wherein the thickening agent and the emulsifier cannot be absent at the same time. The modified milk has not only the functionality of gamma-aminobutyric acid, but also a rich, creamy, and milky flavor as well as smooth texture, and is free of distinctive irritating yeast odor.

## Description

### Technical Field

The present invention relates to the field of modified milk, and in particular, to a modified milk having a yeast extract containing gamma-aminobutyric acid and a preparation method therefor.

### Background Art

There are many liquid milks on the market today, which are often made by adding thickening agents, emulsifiers, sweeteners, etc to the milk.

In recent years, with the acceleration of overall social life rhythm and the impact from all aspects, an increasing number of residents have sleep problems.

With the development of science and technology, scientists have gradually recognized that gamma-aminobutyric acid (GABA) is a non-protein constitutive amino acid widely distributed in animals, plants, and microorganisms, which has physiological activities such as lowering blood pressure, lowering blood sugar, calming nerves, anti-anxiety, sleep aid, and enhancing kidney function. Relevant studies have shown that GABA can bind to anti-anxiety receptors in the brain and activate the enzyme system in the central nervous system. The whole nervous system can cooperate with other substances to prevent the nervous system from receiving anxiety-related information and cut off transmission pathways, thus playing a role in tranquilizing and allaying, anti-anxiety, and sleep aid. By inhibiting the decarboxylation of glutamic acid, GABA can promote more glutamic acid to combine with ammonia to generate urea to be discharged from the body, thereby relieving ammonia toxicity and improving human liver and kidney function. Many clinical trials have shown that gamma-aminobutyric acid has a significant effect on anti-anxiety, sleep aid, and other aspects, and the country has also included it in the list of new resource foods.

### Summary of the Invention

In view of the anti-anxiety, sleep aid, and other effects of gamma-aminobutyric acid, it has been proposed in the prior art to add gamma-aminobutyric acid to liquid milk products to improve sleep quality and inhibit anxiety while ingesting milk nutrition. The inventors have been engaged in yeast research for a long time and have found that the yeast extract prepared by a specific method contains GABA, and the addition of the just-prepared yeast extract containing GABA to liquid milk can meet the realistic needs of consumers.

Accordingly, it is an object of the present invention to provide a modified milk having a yeast extract containing gamma-aminobutyric acid and a preparation method therefor, which can introduce the health function and nutritional value of gamma-aminobutyric acid while improving the mouthfeel and flavor of the product.

In a first aspect, the present invention provides a modified milk, comprising, in a total of 1,000 parts by weight of ingredients: 800-970 parts, 0.1-10 parts of a yeast extract containing gamma-aminobutyric acid, 0-4 parts of a thickening agent, 0-3 parts of an emulsifier, 0-1 parts of an acidity regulator, 0-130 parts of a sweetener, and 0-2 parts of a flavor essence, with the balance being water, wherein the thickening agent and the emulsifier cannot be absent at the same time. Preferably, the modified milk comprises, in a total of 1,000 parts by weight of ingredients: 810-960 parts of raw milk, 0.1-10 parts of a yeast extract containing gamma-aminobutyric acid, 0.28-3.8 parts of a thickening agent, 0.5-3 parts of an emulsifier, 0.3-0.9 parts of an acidity regulator, 10-130 parts of a sweetener, and 0.5-2 parts of a flavor essence, with the balance being water.

Preferably, according to the modified milk above, the raw milk is fresh milk, reconstituted milk, partially skimmed milk, or skimmed milk.

Preferably, according to the modified milk above, the partially skimmed milk or the skimmed milk is obtained by adjusting the fat content of the fresh milk or the reconstituted milk by centrifugation.

Preferably, according to the modified milk above, the yeast extract containing gamma-aminobutyric acid is derived from a yeast fermentation process.

Preferably, the modified milk further comprises one or two or more selected from the group consisting of a nutrient-enhancing substance, a cereal ingredient, a fruit and vegetable ingredient, and a water-soluble dietary fiber;

Preferably, the modified milk further comprises 1-15 parts of a water-soluble dietary fiber; more preferably, the water-soluble dietary fiber is polydextrose.

Preferably, according to the modified milk above, the yeast extract containing gamma-aminobutyric acid is obtained by the yeast fermentation process comprising the steps of:
(1) fermenting yeast to prepare yeast milk, with a yeast inoculation amount of 10 g/L-30 g/L, and adding citric acid with a content of 0.5wt%-5 wt%, preferably 1wt%-2 wt%;
(2) heating the yeast milk obtained in step (1) at a temperature of 40-70°C, preferably 50-60°C;
(3) maintaining the pH of the yeast milk at 6.3 ± 0.2 after heating, and continuing to maintain the temperature for 0.5-3 h, preferably 1-2 h;
(4) after that, controlling the pH at 4.0-7.0, preferably 5.0-6.0, using citric acid;
(5) continuing the autolysis of the yeast milk under the pH condition of step (4) the time of autolysis is 2-8 h, preferably the time of autolysis is 3-5 h; and
(6) separating the yeast milk obtained in step (5) with hot water, evaporating and concentrating the separated clear liquid to obtain a yeast extract containing gamma-aminobutyric acid in a paste state.

Preferably, according to the modified milk above, the thickening agent comprises one or two or more selected from the group consisting of cyclodextrin, gum arabic, acetate starch, guar gum, pectin, sodium alginate, potassium alginate, locust bean gum, xanthan gum, methylcellulose, gellan gum, sodium polyacrylate, carrageenan, distarch phosphate, gelatin, hydroxypropyl starch, carboxypropyl methyl cellulose, agar, sodium carboxymethyl cellulose, microcrystalline cellulose, acetylated distarch phosphate, and acetylated distarch oxalate.

Preferably, according to the modified milk above, the emulsifier comprises one or two or more selected from the group consisting of propylene glycol fatty acid ester, mono-and diglycerides of fatty acids, succinylated monoglyceride, polyglycerol fatty acid ester, polyoxyethylene sorbitan monolaurate, diacetyl tartaric acid ester of mono(di)glycerides, sucrose fatty acid ester, phospholipid, modified soybean phospholipid, enzymatically hydrolyzed soybean phospholipid, sodium caseinate, lactic acid fatty acid glyceride, and acetylated mono-and diglycerides of fatty acids, preferably mono-and diglycerides of fatty acids mixture that may be a mixed ester formed from any fatty acid and glycerol. For example, the mono-and diglycerides of fatty acids in the present invention may be a mixed ester formed from one or two or more selected from the group consisting of oleic acid, linoleic acid, linolenic acid, palmitic acid, behenic acid, stearic acid, and lauric acid, and glycerol; it may be mono-and diglycerol stearate mixture, wherein the mono- and diglycerol fatty acid ester has a monoglycerol fatty acid ester content of 95% or more (monoglycerol stearate are preferred in the present invention). Similarly, the "fatty acid ester" in the "polyglycerol fatty acid ester," the "sucrose fatty acid ester," and the " acetylated mono-and diglycerides of fatty acids" used in the present invention may be any ester formed from any fatty acid and glycerol, sucrose, and acetylated glycerol, respectively. Specifically, the "fatty acid ester" may be one or two or more selected from the group consisting of oleic acid, linoleic acid, linolenic acid, palmitic acid, behenic acid, stearic acid, and lauric acid.

Preferably, according to the modified milk above, the acidity regulator comprises one or two or more selected from the group consisting of phosphoric acid and salts thereof, citric acid and salts thereof, malic acid and salts thereof, lactic acid and salts thereof, carbonic acid and salts thereof. Preferably, according to the modified milk above, the sweetener comprises one or two or more selected from the group consisting of white granulated sugar, glucose-fructose syrup, crystalline fructose, honey, stevioside, erythritol, mogroside, maltitol, sucralose, acesulfame-K, neotame, and sodium cyclamate.

In a second aspect, the present invention provides a method for preparing the modified milk above, comprising the steps of:
(1) dissolving materials comprising a yeast extract containing gamma-aminobutyric acid, a thickening agent and/ an emulsifier, part or all of a sweetener in a part of raw milk to form a mixed material;
(2) homogenizing the mixed material obtained in step (1);
(3) adding the remaining ingredients except for a flavor essence and the remaining raw milk to the mixed material after homogenization treatment;
(4) adding water for constant volume to obtain a semi-finished product, wherein a flavor essence may be added as required; and
(5) sterilizing the semi-finished product to obtain a modified milk.

According to the above method, the content of raw milk used in step (1) accounts for 25wt%-60 wt% of the total content of raw milk.

Preferably, according to the above method, in step (1), the raw milk is heated to dissolve other materials at a temperature of 60-75°C.

Preferably, according to the above method, in step (1), the raw milk is sheared and stirred to dissolve other materials, the time of shearing and stirring is 15-20 min.

Preferably, according to the above method, in step (2), the temperature of homogenization is 60-75°C, and the pressure of homogenization is 70/250 bar.

Moreover, the method further comprises testing the product quality after homogenization and before sterilization, or testing the quality after sterilization; or testing the quality of the semi-finished product obtained in step (2) before homogenization; preferably testing the quality after sterilization, followed by canning to obtain a modified milk.

Specifically, when the quality of the semi-finished product obtained in step (2) is tested, the protein and acidity indicators of the semi-finished product are tested, in which the protein content of the semi-finished product shall be ≥ 2.3%, the acidity shall be 15-18 °T, and the pH value shall be 6.3-7.0.

The modified milk of the present invention can combine excellent mouthfeel and balanced functional nutrition. It has not only the functionality of gamma-aminobutyric acid, but also a rich, creamy, and milky flavor as well as smooth texture, and is free of distinctive irritating yeast odor.

### Detailed Description of the Invention

According to the present invention, the technical problem of both mouthfeel and functional nutrition not being able to be achieved is solved by adding a freshly prepared yeast extract containing gamma-aminobutyric acid directly to raw milk.

The yeast extract containing gamma-aminobutyric acid used in the present invention is prepared by the following steps: fermenting a conventional yeast to prepare yeast milk and adding 0.5%-5 wt%, preferably 1%-2 wt%, of citric acid; then heating the obtained yeast milk at 40°C-70°C, preferably 50-60°C; maintaining the pH of the yeast milk after heating, and continuing to maintain the temperature for 0.5-3 h, preferably 1-2 h; after that, controlling the pH between 4.0 and 7.0, preferably between 5.0 and 6.0, using citric acid; continuing the autolysis of the yeast milk under the above pH condition for 2-8 h, preferably 3-5 h; separating the yeast milk after completion of the reaction with hot water, evaporating and concentrating the separated clear liquid to obtain a yeast extract product containing gamma-aminobutyric acid in a paste state.

Preferably, the following specific yeast strains are used in the present invention to prepare the yeast extract product containing gamma-aminobutyric acid:
*1. Saccharomyces cerevisiae* - strain name: *Saccharomyces cerevisiae* FX-2; accession number: CCTCC NO: M2016418; deposit date: Aug 1, 2016; depository: China Center for Type Culture Collection (CCTCC); address: Wuhan University, Wuhan, China, 430072, fax: (027)68754833; specifically disclosed in the patent literature CN108220175A (application number: 201611141122.8).
*2. Wickerhamomyces anomalus* - strain name: *Wickerhamomyces anomalus* C1.7; accession number: CCTCC NO: M2017782; deposit date: Dec 11, 2017; depository: China Center for Type Culture Collection (CCTCC); address: Wuhan University, Wuhan, China, 430072, fax: (027)68754833; specifically disclosed in the patent literature CN110959853A (application number: 201811152248.4).
3. *Cyberlindnera fabianii* - strain name: *Cyberlindnera fabianii* C1.8; accession number: CCTCC NO: M2017780; deposit date: Dec 11, 2017; depository: China Center for Type Culture Collection (CCTCC); address: Wuhan University, Wuhan, China, 430072, fax: (027)68754833; specifically disclosed in the patent literature CN110959853A (application number: 201811152248.4).

The yeast milk is obtained by the following steps: adding a yeast strain to a sterilized glucose solution, with an inoculation amount of the strain of 15 g/L-30 g/L, and activating to obtain a yeast seed solution; adding the yeast seed solution to a fermentation medium and fermenting to obtain a fermentation broth; centrifuging the fermentation broth and washing the heavy phase to obtain a washed yeast; formulating the washed yeast with water to obtain the yeast milk.

The fermentation conditions are as follows: pH 3.0-5.0, culture temperature 30-33°C, and culture time 10-20 hours.

The fermentation medium contains the following components in mass per 100 milliliters of medium: 3-5 g of a carbon source, 0.5-1 g of a yeast extract, 1-2 g of ammonium sulfate, 0.5-1 g of magnesium sulfate, 0.3-0.5 g of potassium dihydrogen phosphate, and 0.1-0.2 g of zinc sulfate. The carbon source comprises one or two or more selected from the group consisting of cane molasses, beet molasses, and starch hydrolyzing sugars.

The centrifugation of the fermentation broth is performed by centrifuging at 5000-7000 rpm for 5-15 minutes, discarding the supernatant, and retaining the heavy phase.

The washing of the heavy phase is performed by dissolving the heavy phase with water, re-centrifuging, discarding the supernatant, re-dissolving the re-obtained heavy phase with water, centrifuging, and discarding the supernatant to obtain a washed yeast.

The modified milk of the present invention may further be added with one or two or more selected from the group consisting of a nutrient-enhancing substance, a cereal ingredient, a fruit and vegetable ingredient, and a water-soluble dietary fiber, with a total of 1,000 parts by weight of ingredients.

If fresh milk is used as an initial ingredient, the method for preparing a modified milk having a yeast extract containing gamma-aminobutyric acid according to the present invention comprises the steps of:
(1) processing fresh milk by standard processing techniques such as concentration and fat separation to obtain raw milk such as whole milk, partially skimmed milk or skimmed milk, or reconstituted milk;
(2) heating 25%-60% of the total weight of the raw milk in the formula indirectly to 60-75°C, mixing powder ingredients from other ingredients uniformly, slowly adding them to the heated raw milk, maintaining the temperature at 60-75°C, shear stirring until fully mixing uniformly, and adding liquid ingredients (excluding liquid flavor essence) such as syrup after the powder materials are evenly dispersed to obtain a mixed material;
(3) homogenizing the mixed material obtained in step (2) with a homogenization parameter of 70/250 bar (i.e., first adjusting the secondary pressure to 70 bar, and then adjusting the primary pressure to 250 bar) to obtain a semi-finished product;
(4) cooling the semi-finished product obtained in step (3) to 1-8°C, then introducing it into a can to be filled, flushing the pipeline with a small amount of raw milk, and introducing it into the can to be filled;
(5) Introducing the remaining raw milk and water into the can to be filled, and synchronously adding liquid flavor essence;
(6) testing the key indicators of the semi-finished product, in which the protein content shall be ≥ 2.3%, the acidity shall be 15-18 °T, and the pH value shall be 6.3-7.0;
(7) performing an ultra-high temperature sterilization process (130°C-142°C, 3-15 s) to ensure the killing of spores contained in some ingredients while killing conventional microorganisms; and
(8) cooling the material obtained in step (7) to below 35-20°C for canning, wherein the second sterilization process is performed by sterilizing after canning, and the packaging form can be a variety of packaging forms currently on the market for beverage packaging, in particular such as Tetra Prisma, PrePack, Tetra Brik Aseptic Edge, three-piece can, two-piece can, Combibloc, PET, HDPT, etc.

Various indicators of the liquid milk product having a yeast extract containing gamma-aminobutyric acid prepared according to the above process meet the national standard for modified milk.

The technical solution of the present invention will be described in detail with reference to the examples below. Apparently, the described examples are merely some rather than all of the examples of the present invention. All other examples obtained by a person of ordinary skill in the art based on the examples of the present invention without creative efforts shall fall within the protection scope of the present invention.

The yeast extract containing gamma-aminobutyric acid used in all examples is a fresh yeast extract made by fermentation.

The sources of each material used in the following Examples and Comparative Examples are shown in Table A below.

**Table A. Sources of each material used in Examples and Comparative Examples**

| Material name | Model | Vendor and source |
|---|---|---|
| Fresh milk | ---- | Angel Xiwang Food Co., Ltd. |
| White granulated sugar | ---- | Commercial first-class white granulated sugar |
| Polydextrose | Model 90 | Baolingbao Biology |
| mono-and diglycerides of fatty acids | HP-C (total monoglycerol fatty acid ester content ≥ 95% (mass fraction)) | Danisco (China) Co., Ltd. |
| Sucralose | ---- | Anhui Jinhe Industrial Co., Ltd. |
| Food flavor essence | ---- | Dongguan Boton Flavors & Fragrances Co., Ltd. |
| Microcrystalline cellulose (thickening agent) | ---- | Linghu New Hope Chemical Co., Ltd. |
| Disodium hydrogen phosphate | ---- | Xingfa Chemicals Group Co., Ltd. |
| Oat grains | ---- | Yujia Food Co., Ltd. |
| Concentrated strawberry juice | ---- | Xiamen Dachuan Juice Food Co., Ltd. |
| Carrageenan (thickening agent) | CL211 | DuPont Danisco |
| Gellan gum (thickening agent) | GELLANEER-AY | Royal DSM |
| Sodium hexametaphosphate | ---- | Xingfa Chemicals Group Co., Ltd. |
| Gamma-aminobutyric acid | 98% purity | Bloomage Biotech |

### Example 1. Preparation of a yeast extract containing gamma-aminobutyric acid

### 1.1 Preparation of a yeast extract containing gamma-aminobutyric acid:

(1) FX-2 yeast (*Saccharomyces cerevisiae* FX-2) was fermented by molasses to prepare yeast milk, with an inoculation amount of the FX-2 strain of 20 g/L, and 1.5 wt% of citric acid was added;
(2) The yeast milk obtained in step (1) was heated in a water bath at a temperature of 55°C;
(3) The pH of the yeast milk was maintained at 6.3 + 0.2 after heating, and the temperature was continued to be maintained for 0.5 h;
(4) After that, the pH was controlled at 5.5 ± 0.3 using citric acid;
(5) The autolysis of the yeast milk was continued under the pH condition of step (4) for 4 h; and
(6) The yeast milk obtained in step (5) was separated with hot water, and the separated clear liquid was evaporated and concentrated to obtain a yeast extract containing gamma-aminobutyric acid in a paste state for the preparation of modified milk in the following examples, wherein the extract has a solid content of 68%, a gamma-aminobutyric acid content of 10.5% (dry basis), and a protein content of 53% (dry basis).

### Example 2. Preparation of an original flavor modified milk having a yeast extract containing gamma-aminobutyric acid

1. In this example, the ratio of each ingredient was based on the total weight of the modified milk as follows:
   820‰ of fresh milk, 10‰ of white granulated sugar, 10‰ of polydextrose, 10‰ of a yeast extract containing gamma-aminobutyric acid, 1.5‰ of microcrystalline cellulose, 0.5‰ of mono-and diglycerides of fatty acids, 0.06‰ of sucralose, and 1.2‰ of a food flavor essence, with the balance being water.
2. Preparation method
   (1) Fresh milk was processed by standardized processing techniques to obtain raw milk, i.e., the fat of fresh milk was adjusted using a centrifuge, so that the fat content of the adjusted raw milk met the design requirements (the fat content of the raw milk in this example was set as 3%);
   (2) 50% of the total mass of the raw milk was heated to 68°C. White granulated sugar, sucralose, and microcrystalline cellulose were mixed uniformly, added to the heated raw milk, and high-speed sheared for 18 min until there were no visible granules in the mixed liquor. Finally, the yeast extract containing gamma-aminobutyric acid in a paste state was added to obtain a mixed material;
   (3) The mixed material obtained in step (2) was homogenized at a homogenization temperature of 66°C and a homogenization pressure of 70/250 bar, i.e., the secondary pressure was first adjusted to 70 bar, and the primary pressure was then adjusted to 250 bar;
   (4) The mixed material obtained in step (3) was cooled to 5°C using a heat exchange device and then introduced into a can to be filled. The pipeline was flushed with the remaining raw milk, and the part of raw milk was introduced into the can to be filled;
   (5) The volume of the liquor in the can to be filled was adjusted to the ingredient amount using the remaining ingredient water to obtain a semi-finished product;
   (6) The protein and acidity indicators of the semi-finished product were tested, in which the protein mass content was 2.42%, the acidity was 16 °T, and the pH value was 6.8 (the protein content of the semi-finished product shall be ≥ 2.3%, the acidity shall be 15-18 °T, and the pH value shall be 6.3-7.0);
   (7) Two hours before sterilization, the food flavor essence was added to the semi-finished product;
   (8) Ultra-high temperature sterilization
      Vacuum degassing: vacuum degree -70 kpa.
      The pre-homogenization parameter was 50/200 bar, i.e., the secondary pressure was first adjusted to 50 bar, and the primary pressure was then adjusted to 200 bar.
      Tubular sterilization method
      Sterilization parameter: 142°C/5 s.
      Cooled to below 35°C.
   (9) Canning
      The sterile liquor obtained in the process (8) was aseptically canned.
   (10) Finished product inspection
      Various indicators of the finished product were tested according to the steps described in the "Evaluation test method for product mouthfeel" in the following text of the present application. The specific test results are shown in Table 2.

### Example 3. Preparation of an oat flavor modified milk from a yeast extract containing gamma-aminobutyric acid

1. In this example, the ratio of each ingredient was based on the total weight of the modified milk as follows:
   820‰ of fresh milk, 10‰ of white granulated sugar, 10‰ of polydextrose, 5‰ of a yeast extract containing gamma-aminobutyric acid, 2.0‰ of microcrystalline cellulose, 0.3‰ of disodium hydrogen phosphate, 5‰ of oat grains, 0.06‰ of sucralose, and 1.2‰ of a food flavor essence, with the balance being water.
2. Preparation method
   (1) Fresh milk was processed by standardized processing techniques to obtain raw milk, i.e., the fat of fresh milk was adjusted using a centrifuge, so that the fat content of the adjusted raw milk met the design requirements (the fat content of the raw milk in this example was set as 3%);
   (2) 45% of the total mass of the raw milk was heated to 65°C. White granulated sugar, sucralose, and microcrystalline cellulose were mixed uniformly, added to the heated raw milk, and high-speed shear stirred for 15 min until there were no visible granules in the mixed liquor. Finally, the yeast extract containing gamma-aminobutyric acid in a paste state was added to obtain a mixed material;
   (3) The mixed material obtained in step (2) was homogenized at a homogenization temperature of 65°C and a homogenization pressure of 70/250 bar, i.e., the secondary pressure was first adjusted to 70 bar, and the primary pressure was then adjusted to 250 bar;
   (4) The mixed material obtained in step (3) was cooled to 1-10°C using a heat exchange device and then introduced into a can to be filled. The pipeline was flushed with the remaining raw milk, and the part of raw milk was introduced into the can to be filled;
   (5) The oat grains were soaked in water at normal temperature at a ratio of soaking material to water of 1:4 (mass ratio) for 50 min, and then cooked and softened in water at 75°C for 15 min until the oat grains had no hard core;
   (6) The volume of the liquor in the can to be filled was adjusted to the ingredient amount using the remaining ingredient water to obtain a semi-finished product;
   (9) The protein and acidity indicators of the semi-finished product were tested, in which the protein mass content was 2.50%, the acidity was 15 °T, and the pH value was 6.5 (the protein content of the semi-finished product shall be ≥ 2.3%, the acidity shall be 15-18 °T, and the pH value shall be 6.3-7.0);
   (7) Two hours before sterilization, the food flavor essence was added to the semi-finished product;
   (8) The pre-processed oat grains were mixed with the semi-finished product in-line through a dynamic mixing device and then subjected to a sterilization process;
   (9) Ultra-high temperature sterilization
      Vacuum degassing: vacuum degree -35 kpa.
      The pre-homogenization parameter was 50/200 bar, i.e., the secondary pressure was first adjusted to 50 bar, and the secondary pressure was then adjusted to 200 bar.
      Tubular sterilization method
      Sterilization parameter: 142°C/5 s. Cooled to below 35°C.
      (11) Canning
         The sterile liquor obtained in the process (10) was aseptically canned.
      (12) Finished product inspection
         Various indicators of the finished product were tested according to the steps described in the " Evaluation test method for product mouthfeel" in the following text of the present application. The specific test results are shown in Table 2.

### Example 4. Preparation of a strawberry flavor modified milk from a yeast extract containing gamma-aminobutyric acid

1. In this example, the ratio of each ingredient was based on the total weight of the modified milk as follows:
   810‰ of fresh milk, 15‰ of white granulated sugar, 5‰ of concentrated strawberry juice, 2.5‰ of a yeast extract containing gamma-aminobutyric acid, 2.0‰ of microcrystalline cellulose, 1.2‰ of mono-and diglycerides of fatty acids, 0.16‰ of carrageenan, 0.12‰ of gellan gum, 0.2‰ of sodium hexametaphosphate, 0.3‰ of disodium hydrogen phosphate, 0.18‰ of sucralose, and 1.5‰ of a food flavor essence, with the balance being water.
2. Method
   (1) Fresh milk was processed by standardized processing techniques to obtain raw milk, i.e., the fat of fresh milk was adjusted using a centrifuge, so that the fat content of the adjusted raw milk met the design requirements (the fat content of the raw milk in this example was set as 3%);
   (2) 50% of the total amount of the raw milk was heated to 70°C. White granulated sugar, the yeast extract, microcrystalline cellulose, mono-and diglycerides of fatty acids, carrageenan, and gellan gum were mixed uniformly, added to the heated raw milk, and high-speed shear stirred for 20 min until there were no visible granules in the mixed liquor, so as to obtain a mixed material;
   (3) The concentrated strawberry juice, disodium hydrogen phosphate, and sodium hexametaphosphate were dissolved and mixed in water with a mass concentration of 10% to obtain a mixed material;
   (4) The mixed materials obtained in steps (2) and (3) were mixed, high shear stirred for 10 min, and homogenized at a homogenization temperature of 70°C and a homogenization pressure of 70/250 bar, i.e., the secondary pressure was first adjusted to 70 bar, and the primary pressure was then adjusted to 250 bar;
   (5) The mixed material obtained in step (4) was cooled to 10°C using a heat exchange device and then introduced into a can to be filled. The pipeline was flushed with the remaining milk, and the part of the milk was introduced into the can to be filled;
   (6) The volume of the liquor in the can to be filled was adjusted to the ingredient amount using the remaining ingredient water to obtain a semi-finished product; the protein and pH indicators of the semi-finished product were tested, in which the protein mass content was 2.40%, the acidity was 16 °T, and the pH value was 6.5 (the protein content of the semi-finished product shall be ≥ 2.3%, the acidity shall be 15-18 °T, and the pH value shall be 6.3-7.0);
   (7) Two hours before sterilization, the food flavor essence was added;
   (8) Ultra-high temperature sterilization
      Vacuum degassing: vacuum degree -75 kpa.
      The pre-homogenization parameter was 50/200 bar, i.e., the secondary pressure was first adjusted to 50 bar, and the secondary pressure was then adjusted to 200 bar.
      Tubular sterilization method
      Sterilization parameter: 138°C/3 s.
      Cooled to below 35°C.
   (9) Canning
      The sterile liquor obtained in the process (8) was aseptically canned.
   (10) Finished product inspection
      Various indicators of the finished product were tested according to the steps described in the " Evaluation test method for product mouthfeel " in the following text of the present application. The specific test results are shown in Table 2.

### Example 5. Preparation of a strawberry flavor modified milk from a yeast extract containing gamma-aminobutyric acid

In this example, the ratio of each ingredient was based on the total weight of the modified milk as follows:
810‰ of fresh milk, 125‰ of white granulated sugar, 5‰ of concentrated strawberry juice, 2.5‰ of a yeast extract containing gamma-aminobutyric acid, 2.0‰ of microcrystalline cellulose, 2.8‰ of mono-and diglycerides of fatty acids, 0.16‰ of carrageenan, 0.12‰ of gellan gum, 0.2‰ of sodium hexametaphosphate, 0.3‰ of disodium hydrogen phosphate, 0.18‰ of sucralose, and 1.5‰ of a food flavor essence, with the balance being water.

Except that the specific content of each component involved was different from those in Example 4 as described above, the modified milk was prepared by using the same operation steps as in Section 4.2 of Example 4. Various indicators of the prepared modified milk finished product were tested according to the steps described in the " Evaluation test method for product mouthfeel" in the following text of the present application. The specific test results are shown in Table 2.

### Example 6. Preparation of an oat flavor modified milk from a yeast extract containing gamma-aminobutyric acid

In this example, the ratio of each ingredient was based on the total weight of the modified milk as follows:
820‰ of fresh milk, 10‰ of white granulated sugar, 10‰ of polydextrose, 5‰ of a yeast extract containing gamma-aminobutyric acid, 3.8‰ of microcrystalline cellulose, 0.9‰ of disodium hydrogen phosphate, 5‰ of oat grains, 0.06‰ of sucralose, and 1.2‰ of a food flavor essence, with the balance being water.

Except that the specific content of each component involved was different from those in Example 3 as described above, the modified milk was prepared by using the same operation steps as in Section 3.2 of Example 3. Various indicators of the prepared modified milk finished product were tested according to the steps described in the " Evaluation test method for product mouthfeel" in the following text of the present application. The specific test results are shown in Table 2.

### Example 7. Preparation of an original flavor modified milk from a yeast extract containing gamma-aminobutyric acid

In this example, the ratio of each ingredient was based on the total weight of the modified milk as follows:
960‰ of fresh milk, 10‰ of white granulated sugar, 10‰ of polydextrose, 10‰ of a yeast extract containing gamma-aminobutyric acid, 1.5‰ of microcrystalline cellulose, 0.5‰ of mono-and diglycerides of fatty acids, 0.06‰ of sucralose, and 1.2‰ of a food flavor essence, with the balance being water.

Except that the specific content of each component involved was different from those in Example 2 as described above, the modified milk was prepared by using the same operation steps as in Section 2.2 of Example 2, in which the material dissolution process was performed by dissolving the thickening agent, the emulsifier, the sweetener, and the acidity regulator in fresh milk. Various indicators of the prepared modified milk finished product were tested according to the steps described in the " Evaluation test method for product mouthfeel" in the following text of the present application. The specific test results are shown in Table 2.

### Example 8. Preparation of an original flavor modified milk from a yeast extract containing gamma-aminobutyric acid

### Preparation of a modified milk

In this example, the ratio of each ingredient was based on the total weight of the modified milk as follows:
820‰ of fresh milk, 10‰ of white granulated sugar, 10‰ of polydextrose, 0.08‰ of a yeast extract containing gamma-aminobutyric acid, 1.5‰ of microcrystalline cellulose, 0.5‰ of mono-and diglycerides of fatty acids, 0.06‰ of sucralose, and 1.2‰ of a food flavor essence, with the balance being water.

Except that the specific content of each component involved was different from those in Example 1 as described above, the modified milk was prepared by using the same operation steps as in Section 2.2 of Example 2. Various indicators of the prepared modified milk finished product were tested according to the steps described in the " Evaluation test method for product mouthfeel" in the following text of the present application. The specific test results are shown in Table 2.

### Example 9. Preparation of an original flavor modified milk from a yeast extract containing gamma-aminobutyric acid Preparation of a modified milk

In this example, the ratio of each ingredient was based on the total weight of the modified milk as follows:
820‰ of fresh milk, 10‰ of white granulated sugar, 10‰ of polydextrose, 12‰ of a yeast extract containing gamma-aminobutyric acid, 1.5‰ of microcrystalline cellulose, 0.5‰ of mono-and diglycerides of fatty acids, 0.06‰ of sucralose, and 1.2‰ of a food flavor essence, with the balance being water. Except that the specific content of each component involved was different from those in Example 1 as described above, the modified milk was prepared by using the same operation steps as in Section 2.2 of Example 2. Various indicators of the prepared modified milk finished product were tested according to the steps described in the " Evaluation test method for product mouthfeel" in the following text of the present application. The specific test results are shown in Table 2.

### Comparative Example 1. Preparation of an original flavor modified milk from pure gamma-aminobutyric acid

In this example, the ratio of each ingredient was based on the total weight of the modified milk as follows:
1. Formula: 820‰ of fresh milk, 10‰ of white granulated sugar, 10‰ of polydextrose, 0.73‰ of gamma-aminobutyric acid, 0.5‰ of mono-and diglycerides of fatty acids, 0.06‰ of sucralose, and 1.2‰ of a food flavor essence, with the balance being water.
2. Preparation method
   (1) Fresh milk was processed by standardized processing techniques to obtain raw milk, i.e., the fat of fresh milk was adjusted using a centrifuge, so that the fat content of the adjusted raw milk met the design requirements (the fat content of the raw milk in this example was set as 3%);
   (2) 40-60 wt% of the total amount of the raw milk was heated to 65-70°C. White granulated sugar, sucralose, and microcrystalline cellulose were mixed uniformly, added to the heated raw milk, and high-speed sheared for 15-20 min until there were no visible granules in the mixed liquor. Finally, gamma-aminobutyric acid was added to obtain a mixed material;
   (3) The mixed material obtained in step (2) was homogenized at a homogenization temperature of 65-70°C and a homogenization pressure of 70/250 bar, i.e., the secondary pressure was first adjusted to 70 bar, and the primary pressure was then adjusted to 250 bar;
   (4) The mixed material obtained in step (3) was cooled to 1-10°C using a heat exchange device and then introduced into a can to be filled. The pipeline was flushed with the remaining raw milk, and the part of raw milk was introduced into the can to be filled;
   (5) The volume of the liquor in the can to be filled was adjusted to the ingredient amount using the remaining ingredient water to obtain a semi-finished product;
   (6) The protein and acidity indicators of the semi-finished product were tested;
   (7) Two hours before sterilization, the food flavor essence was added to the semi-finished product;
   (8) Ultra-high temperature sterilization
      Vacuum degassing: vacuum degree -35 to -80 kpa.
      The pre-homogenization parameter was 50/200 bar, i.e., the secondary pressure was first adjusted to 50 bar, and the secondary pressure was then adjusted to 200 bar.
      Tubular sterilization method
      Sterilization parameter: 142°C/5 s.
      Cooled to below 35°C.
   (9) Canning
      The sterile liquor obtained in step (8) was aseptically canned.
   (10) Finished product inspection
      Various indicators of the finished product were tested.

Evaluation test method for product mouthfeel:
Fifteen professional evaluators were selected to taste the modified milk of Examples 1-3 and Comparative Example 1. The sensory content included odor, taste, appearance, and mouthfeel. The evaluation criteria are shown in Table 1, and the evaluation results are shown in Table 2.

**Table 1**

| Evaluation item | Characteristics | Score |
|---|---|---|
| Appearance | Milky white or nearly milky white emulsion, no abnormal color, no fat floating, no layering and water separation phenomenon | 1-10 |
| Odor | With characteristic flavors such as milky flavor, oat aroma, and strawberry aroma | 1-10 |
| Taste | No peculiar taste | 1-10 |
| Mouthfeel | Smooth and non-sticky | 1-10 |
| Grain texture | Moderate hardness, no damage to teeth, granules remain relatively intact | 1-10 |

**Table 2**

| Sample | Appearance | Odor | Taste | Mouthfeel | Grain texture | Overall sensory description |
|---|---|---|---|---|---|---|
| Example 2 | 8 | 8 | 7 | 7 | -- | Light milky flavor, no peculiar taste |
| Example 3 | 8 | 8 | 7.5 | 7.5 | 8 | Light milky flavor, with oat flavor, smooth mouthfeel, moderate oat granule hardness |
| Example 4 | 8 | 8 | 7 | 8 | -- | Light milky flavor, moderate strawberry flavor, smooth overall mouthfeel |
| Example 5 | 8 | 7 | 6.5 | 6 | -- | Light milky flavor, moderate strawberry flavor, slightly greasy, not refreshing enough |
| Example 6 | 6 | 7 | 6 | 5 | 8 | Too thick overall mouthfeel, not smooth enough, poor overall mouthfeel |
| Example 7 | 8 | 8.5 | 7 | 7.5 | -- | The milky flavor was superior to Example 2, with a thicker and smoother taste. |
| Example 8 | 8 | 8 | 6 | 5 | -- | Light milky flavor, no peculiar taste, but overall bland |
| Example 9 | 8 | 6 | 4 | 5 | -- | With a peculiar taste, distinct yeast flavor |
| Comparative Example 1 | 8 | 7 | 6 | 6 | -- | Light milky flavor, thin mouthfeel, not mellow, relatively smooth |

## Claims

1. A modified milk, comprising, in a total of 1,000 parts by weight of ingredients: 800-970 parts of raw milk, 0.1-10 parts of a yeast extract containing γ-aminobutyric acid, 0-4 parts of a thickening agent, 0-3 parts of an emulsifier, 0-1 parts of an acidity regulator, 0-130 parts of a sweetener, and 0-2 parts of a flavor essence, with the balance being water, wherein the thickening agent and the emulsifier cannot be absent at the same time.

2. The modified milk according to claim 1, comprising, in a total of 1,000 parts by weight of ingredients: 810-960 parts of raw milk, 0.1-10 parts of a yeast extract containing γ-aminobutyric acid, 0.28-3.8 parts of a thickening agent, 0.5-3 parts of an emulsifier, 0.3-0.9 parts of an acidity regulator, 10-130 parts of a sweetener, and 0.5-2 parts of a flavor essence, with the balance being water.

3. The modified milk according to claims 1 or 2, wherein the raw milk is fresh milk, reconstituted milk, partially skimmed milk, or skimmed milk.

4. The modified milk according to claim 3, wherein the partially skimmed milk or the skimmed milk is obtained by adjusting the fat content of the fresh milk or the reconstituted milk by centrifugation.

5. The modified milk according to any one of claims 1-4, wherein the modified milk further comprises one or two or more selected from the group consisting of a nutrient-enhancing substance, a cereal ingredient, a fruit and vegetable ingredient, and a water-soluble dietary fiber; preferably, the modified milk further comprises 1-15 parts of the water-soluble dietary fiber; more preferably, the water-soluble dietary fiber is polydextrose.

6. The modified milk according to any one of claims 1-5, wherein the yeast extract containing γ-aminobutyric acid is derived from a yeast fermentation process.

7. The modified milk according to any of claims 1-6, wherein the yeast extract containing γ-aminobutyric acid is obtained by the yeast fermentation process comprising the steps of:
(1) fermenting yeast to prepare yeast milk, with a yeast inoculation amount of 10-30 g/L, and adding citric acid with a content of 0.5wt%-5 wt%, preferably 1wt%-2 wt%;
(2) heating the yeast milk obtained in step (1) at a temperature of 40-70°C, preferably 50-60°C;
(3) maintaining the pH of the yeast milk at 6.3 ± 0.2 after heating, and continuing to maintain the temperature for 0.5-3 h, preferably 1-2 h;
(4) after that, controlling the pH at 4.0-7.0, preferably 5.0-6.0, using citric acid;
(5) continuing the autolysis of the yeast milk under the pH condition of step (4), preferably the time of autolysis is 2-8 h, more preferably the time of autolysis is 3-5 h; and
(6) separating the yeast milk obtained in step (5) with hot water, evaporating and concentrating the separated clear liquid to obtain a yeast extract containing γ-aminobutyric acid in a paste state.

8. The modified milk according to any one of claims 1-7, wherein the thickening agent comprises one or two or more selected from the group consisting of cyclodextrin, gum arabic, acetate starch, guar gum, pectin, sodium alginate, potassium alginate, locust bean gum, xanthan gum, methylcellulose, gellan gum, sodium polyacrylate, carrageenan, distarch phosphate, gelatin, hydroxypropyl starch, carboxypropyl methyl cellulose, agar, sodium carboxymethyl cellulose, microcrystalline cellulose, acetylated distarch phosphate, and acetylated distarch oxalate.

9. The modified milk according to any one of claims 1-8, wherein the emulsifier comprises one or two or more selected from the group consisting of propylene glycol fatty acid ester, mono-and diglycerides of fatty acids, succinylated monoglyceride, polyglycerol fatty acid ester, polyoxyethylene sorbitan monolaurate, diacetyl tartaric acid ester of mono(di)glycerides, sucrose fatty acid ester, phospholipid, modified soybean phospholipid, enzymatically hydrolyzed soybean phospholipid, sodium caseinate, lactic acid fatty acid glyceride, and acetylated mono-and diglycerides of fatty acids.

10. The modified milk according to any one of claims 1-9, wherein the acidity regulator comprises one or two or more selected from the group consisting of phosphoric acid and salts thereof, citric acid and salts thereof, malic acid and salts thereof, lactic acid and salts thereof, carbonic acid and salts thereof.

11. The modified milk according to any one of claims 1-10, wherein the sweetener comprises one or two or more selected from the group consisting of white granulated sugar, glucose-fructose syrup, crystalline fructose, honey, stevioside, erythritol, mogroside, maltitol, sucralose, acesulfame-K, neotame, and sodium cyclamate.

12. A method for preparing the modified milk according to any one of claims 1-11, comprising the steps of:
(1) dissolving materials comprising a yeast extract containing γ-aminobutyric acid, a thickening agent and/ an emulsifier, part or all of a sweetener in a part of raw milk to form a mixed material;
(2) homogenizing the mixed material obtained in step (1);
(3) adding the remaining ingredients except for a flavor essence and the remaining raw milk to the mixed material after homogenization treatment;
(4) adding water for constant volume to obtain a semi-finished product, wherein the flavor essence may be added or not added as required during adding water for constant volume to obtain a semi-finished product; and
(5) sterilizing the semi-finished product to obtain the modified milk.

13. The method according to claim 12, wherein the content of raw milk used in step (1) accounts for 25wt%-60 wt% of the total content of raw milk.

14. The method according to claims 12 or 13, wherein in step (1), the raw milk is heated to dissolve other materials at a temperature of 60-75°C.

15. The method according to any one of claims 12-14, wherein in step (1), the raw milk is sheared and stirred to dissolve other materials, preferably the time of shearing and stirring is 15-20 min.

16. The method according to any one of claims 12-15, wherein in step (2), the temperature of homogenization is 60-75°C, and the pressure of homogenization is 70/250 bar.

17. The method according to any one of claims 12-16, wherein the method further comprises testing the product quality after homogenization and before sterilization, or testing the quality after sterilization; or testing the quality of the semi-finished product obtained in step (2) before homogenization; preferably testing the quality after sterilization, followed by canning to obtain a modified milk.

18. A modified milk prepared by the method according to any one of claims 12-17.
